# EUROPÄISCHE PATENTSCHRIFT

(11) **EP 0 535 059 B1**
(45) Veröffentlichungstag und Bekanntmachung des Hinweises auf die Patenterteilung: **29.11.1995**
(21) Anmeldenummer: 91911228.4
(22) Anmeldetag: 20.06.1991
(51) Int. Cl.: C12N 15/57

(54) **DNA-SEQUENZ FÜR EINE SERIN-PROTEASE UND DAMIT ZUSAMMENHÄNGENDE GEGENSTÄNDE**
DNA SEQUENCE FOR A SERINE PROTEASE AND ASSOCIATED ITEMS
SEQUENCE D'ADN POUR UNE PROTEASE DE SERINE ET OBJETS S'Y RAPPORTANT

(30) Priorität: 22.06.1990 DE 4019984
(43) Veröffentlichungstag der Anmeldung: 07.04.1993
(73) Patentinhaber: LÜDEMANN, Jens, D-24105 Kiel (DE); UTECHT, Bert, D-24211 Rastorf (DE)
(72) Erfinder: JENNE, Dieter, E., CH-1066 Epalinges (CH); TSCHOPP, Jürg, CH-1066 Epalinges (CH); LÜDEMANN, Jens, CH-1066 Epalinges (CH); UTECHT, Bert, CH-1066 Epalinges (CH); GROSS, Wolfgang, L., CH-1066 Epalinges (CH)
(74) Vertreter: Boeters, Hans Dietrich, Dr.
(86) Internationale Anmeldenummer: EP9101142
(87) Internationale Veröffentlichungsnummer: WO9200378

(56) Entgegenhaltungen:
- NATURE, Band 346, 9. August 1990, Seite 520, (London, GB), D.E. JENNE et al.: "Wegener's autoantigen decoded", siehe den ganzen Artikel
- BLOOD, Band 74, Nr. 6, 1. November 1989, Seiten 1888-1893, (New York, US), J.L. NILES et al.: "Wegener's granulomatosis autoantigen is a novel neutrophil serine proteinase", siehe Seite 1889, linke Spalte, Zeilen 6-16; Seite 1890, linke Spalte, Zeilen 10-23
- SCIENCE, Band 239, 29. Januar 1988, Seiten 487-491, (Lancaster, PA, US), R.K. SAIKI et al.:
- SAIKI et al.: "Primer-directed enzymatic amplification of DNA with a thermostable DNA polymerase", siehe Seite 491, linke Spalte, Zeilen 23-37

## Beschreibung

Die Erfindung betrifft ein in den azurophilen Granula von neutrophilen Granulozyten und in zytoplasmatischen Granula von Monozyten lokalisiertes Autoantigen (Proteinase 3). Die Proteinase 3 (PR-3) ist das Zielantigen der sogenannten antizytoplasmatischen Antikörper (ACPA; Synonyme: "anti-neutrophil cytoplasm antibodies" = ANCA bzw. "cytoplasmic pattern" ANCA = C-ANCA), die eine hohe Spezifität für die Wegener'sche Granulomatose (WG) haben. Darüberhinaus besteht eine enge Korrelation zwischen der Höhe der Antikörper-Titer und der Krankheitsaktivität. Die WG wird dem Formenkreis der primären Vaskulitiden zugeordnet und stellt wegen des sehr variablen Krankheitsbildes eine Herausforderung an verschiedene Fachdisziplinen hinsichtlich Diagnostik und Therapie dar. Die Detektion bzw. Quantifizierung von ACPA ist somit von großer Bedeutung sowohl für die Diagnosefindung als auch für die Verlaufskontrolle der WG; vergleiche in Nölle et al., Ann. Int. Med., 111 (1989) 28-40.

Proteinase 3 wurde bereits aus menschlichen Granulozyten gesunder Spender in größerer Menge durch Affinitätschromatographie mit Immunglobulinen aus Patientenseren gereinigt; vergleiche Lüdemann et al. in J. Exp. Med., 171 (1990) 357-362. Nach diesem Stand der Technik wurde die gereinigte Proteinase 3 auch nach dem Edmann'schen Abbauverfahren amino-terminal ansequenziert. Ein abweichendes Ansequenzierungsergebnis wird von Niles et al. in Blood 74 (1989) 1888 - 1893 berichtet.

Es besteht jedoch ein Bedürfnis, Proteinase 3 in größeren Mengen zur Verfügung zu stellen, einerseits zur Präzisierung der Diagnostik oder für die Therapie und andererseits zur Modifikation in Hinblick auf eine Therapieverbesserung.

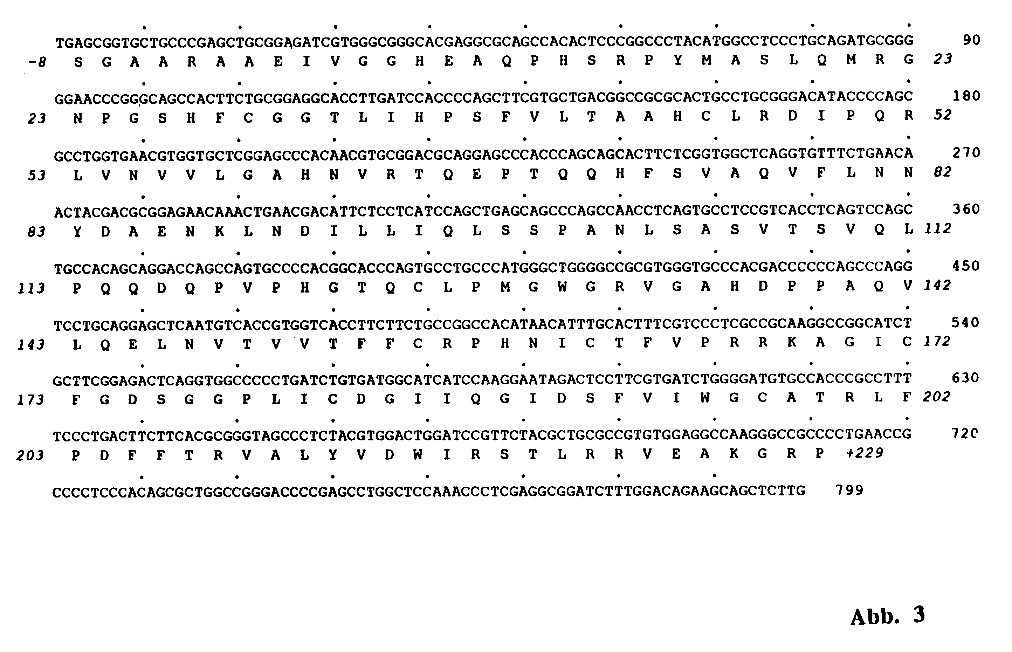

Erfindungsgemäß wird nun eine sDNA vorgesehen, die ein Enzym mit Serinprotease-Aktivität (Proteinase 3) kodiert und ***gekennzeichnet*** ist durch (in Leserichtung)
(a) fakultative Kodons für ein Signalpeptid von Serinprotease,
(b) 2 fakultative Kodons für ein Propeptid von Serinprotease und
(c) 229 Kodons für das prozessierte Enzym mit Serinprotease aktivität (1 bis 229) gemäß Figur 3, wobei beim Merkmal (c)
   - 1 bis 10 oder bis 20 Kodons gemäß Figur 3 gegen andere Kodons ausgetauscht sein können und/oder
   - 1 bis 10 oder bis 20 Kodons fehlen oder hinzugefügt sein können.

Bei der erfindungsgemäßen sDNA können beim Merkmal (c) am 5'-Ende für das prozessierte Enzym mit Serinprotease aktivität 1 bis 10 oder bis 20 Kodons eingefügt sein.

Die erfindsgemäße sDNA kann dadurch ***gekennzeichnet*** sein, daß es sich bei den Kodons für das Signalpeptid (-8 bis -3) um die Kodons gemäß Figur 3 oder eine vom 5'-terminalen Ende her um ein oder mehrere Kodons verkürzte DNA-Sequenz handelt.

Bei der erfindungsgemäßen sDNA kann es sich bei den 2 Kodons für ein Propeptid von Serinprotease (Proteinase 3) um die Kodons handeln, die den Aminosäuresequenz-Positionen -2 bis -1 in Figur 3 entsprechen.

Bei der erfindungsgemäßen sDNA können ein oder mehrere Kodons gemäß Figur 3 durch Kodons ersetzt sein, die dieselbe Aminosäure kodieren.

Erfindungsgemäß wird ferner eine cDNA vorgesehen, bei der es sich um eine erfindungsgemäße sDNA handelt, die um einen komplementären Strang ergänzt ist.

Erfindungsgemäß wird schließlich der Verwendung einer erfindungsgemäßen cDNA, ergänzt um einen komplementären Strang (cDNA), für oder in einem Expressionsvektor zur Expression eines Enzyms mit Serinprotease-Aktivität in Mikroorganismen, insbesondere in E. coli vorgesehen, beispielsweise in pGEX-2T. Ebenso wird erfindungsgemäß die Verwendung einer cDNA, ergänzt um einen komplementären Strang (cDNA), für oder in einer eukaryontischen Zell-Linie zur Expression eines Enzyms mit Serinprotease-Aktivität vorgesehen.

Nachstehend werden die vorstehend angesprochenen Ausführungsformen der Erfindung anhand von Abbildungen näher erläutert. Es zeigen:
Abb. 1 PCR-Produkte;
Abb. 2 Nukleotid- und Aminosäure-Sequenzen der Proteinase 3 (PR-3);
Abb. 3 Nukleotidbasensequenz der cDNA für Proteinase 3 und die davon abgeleitete Proteinsequenz;
Abb. 4 PCR-Produkte.

Bei der Erfindung zugrundeliegenden Arbeiten wurden die ersten einundzwanzig amino-terminalen Aminosäuren mit den im Stand der Technik publizierten und von cDNA-Sequenzen abgeleiteten Aminosäure-Sequenzen verglichen. Dabei ergab sich der Hinweis, daß Proteinase 3 zur Familie der Serin-Proteasen gehört und daß Proteinase 3 zwar große Ähnlichkeiten mit der von einer cDNA-Sequenz abgeleiteten Myeloblastin-Sequenz (Bories et al., Cell, 59 (1989) 959-968) aufweist, am Amino-Terminus jedoch eindeutig von der publizierten Sequenz des Myeloblastins abweicht.

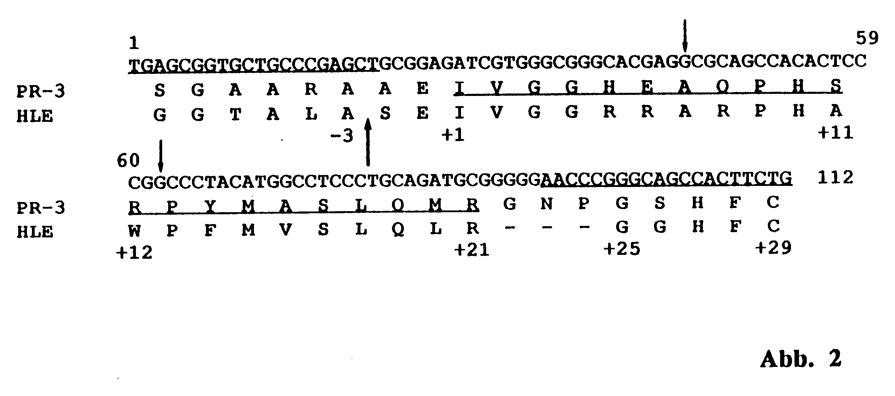

Insbesondere fiel bei diesen der Erfindung zugrundeliegenden Arbeiten auf, daß die ersten sieben Aminosäuren von Myeloblastin mit den erfindungsgemäß ermittelten Aminosäureresten 15 bis 21 der Proteinase 3 übereinstimmen. Ferner wurde erfindungsgemäß festgestellt, daß die publizierten 66 Nukleotidbasen des nicht-kodierenden 5'-Endes der Myeloblastin-cDNA-Sequenz nicht die genetische Information für die ersten 14 Aminosäuren des Wegenerschen Autoantigens darstellen. Aus diesem Grunde wurden bei den der Erfindung zugrundeliegenden Arbeiten die 5'-cDNA-Region der Position -66 bis +44 (siehe Bories et al., Cell, 59 (1989) 959-968) unter Verwendung der PCR-Technik analysiert. Mit Hilfe von zwei Oligonukleotiden und der TAQ-DNA-Polymerase wurde ein 112 Nukleotidbasen langes cDNA-Fragment aus totaler U937-cDNA herausamplifiziert und nach Subklonierung in M13mp18 sequenziert. Die so ermittelte cDNA-Sequenz der 5'-Region kodiert den Amino-Terminus der Proteinase 3 (**Abb.2**).

Die erfindungsgemäß ermittelte cDNA-Sequenz der 5'-Region weist an zwei Positionen Unterschiede zur cDNA-Sequenz des Myeloblastins (**Abb.2**) auf. In Postion 45 und 62 findet sich jeweils ein G, das in der Myeloblastin-cDNA-Sequenz fehlt. Dadurch ändert sich der offene Leserahmen in der 5'-cDNA-Region. Dieser neue Leserahmen reicht nun bis zur ersten Base der Nukleotidbasensequenz und enthält neben der vollständigen genetischen Information für die prozessierte sekretierte Form der Proteinase 3 auch die genetische Information für einen Teil des Signalpeptides, das bei der Translokation des Protein-Precursors in das rauhe endoplasmatische Retikulum proteolytisch entfernt wird, ein 2 Amonisäuren langes Propetid, das während des intrazellulären Transports in die zytoplasmatischen Granula abgespalten wird, und die genetische Information für den funktionell wichtigen Amino-Terminus des Enzyms in seiner aktiven, sekretierbaren Form. Vergleicht man die durch Aminosäuresequenzierung gewonnene PR-3-Sequenz von Position 1 bis 21 mit der von der cDNA-Sequenz abgeleiteten Aminosäure-Sequenz, so wird klar, daß die erfindungsgemäß in der 5'-Region bestimmte cDNA-Sequenz (Positionen 21 bis 91; vergleiche **Abb.2**) in Verbindung mit der für Myeloblastin publizierten cDNA-Sequenz ab Nukleotidposition +25 (Bories et al., Cell, 59 (1989) 959-968) die genetische Information für Proteinase 3 (Wegener'sches Autoantigen) korrekt wiedergibt (**Abb.3**).

Durch die Beschreibung der vollständigen cDNA-Sequenz für Proteinase 3 ist es nunmehr mit Hilfe der PCR-Technik möglich, die komplette kodierende cDNA für Proteinase 3 aus U937-cDNA mit Hilfe der Oligonukleotid-Primer JT 88 und JT 90 (5'-CCGAATTC GAGGGTTTGGAGCCAGGCTC-3') zu amplifizieren (**Abb.3**) und in bakterielle Vektoren zu klonieren. Nach Modifikation des 5'-cDNA-Endes durch gezielte Mutagenese kann die Proenzym-Form und die aktive Form des Enzyms in rekombinanter Form in unbegrenzter Menge hergestellt und für wissenschaftliche Studien sowie biomedizinische Anwendungen verfügbar gemacht werden. Ferner lassen sich Fragmente der Proteinase 3 allein oder in Verknüpfung mit anderen Protein-Anteilen auf rekombinantem Wege produzieren.

Weitere Ausführungsformen der Erfindung betreffen schließlich die Verwendung der erfindungsgemäßen Serinprotease zur Gewinnung und/oder Ermittlung von Antikörpern, insbesondere bei der Wegener'schen Granulomatose, oder für die orale oder parenterale Antigen-Therapie bei der Wegener'schen Granulomatrose oder bei Autoimmunreaktionen, die durch Autoantikörperbildung gegen Serinprotease ***gekennzeichnet*** sind.

Für diese Ausführungsformen der Erfindung ist folgendes von Bedeutung: Proteinase 3 wird in den azurophilen (d. h. primären) Granula von neutrophilen Granulozyten und in zytoplasmatischen Granula von Monozyten gespeichert. Die Translation dar mRNA und Produktion von PR-3 erfolgt jedoch hauptsächlich in den Vorstufen der Monozyten und Granulozyten. Proteinase 3 wird mit der Leukozyten-Elastase, dem Cathepsin G und einer Reihe weiterer Enzyme nach Stimulation der Granulozyten, beispielsweise durch Immunkomplexe, chemotaktische Faktoren oder bakterielle Komponenten, in das perizelluläre Milieu abgegeben. Die Bildung von Sauerstoffradikalen, aber auch die Wirkung der neutralen Proteasen verursachen Gewebedestruktionen und Katabolismus der extrazellulären Matrix. Kollagenasen, Gelatinase, Cathepsin G, Leukozyten-Elastase und Proteinase 3 sind imstande, die Schlüsselkomponenten der extrazellulären Matrix, Kollagen, Elastin und Proteoglykane abzubauen. Dieser Abbau erfolgt in der unmittelbaren perizellulären Umgebung der neutrophilen Granulozyten, während die schädigende Fernwirkung der Granulozyten-Proteasen durch Plasma-Inhibitoren effizient verhindert wird. Inaktivierung der Leukozyten-Elastase und Proteinase 3 durch α-1-Protease-Inhibitor unterbleibt in Membrannähe, da höchstwahrscheinlich der α-1-Protease-Inhibitor durch Sauerstoff-Radikale inaktiviert wird. In Tierversuchen wurde gezeigt, daß sich nach einmaliger intertrachealer Injektion von 0,1 mg Proteinase 3 im Verlaufe von 10 Wochen ein ausgeprägtes generalisiertes bullöses Emphysem in der Lunge der Tiere entwickelt; vergl. Kao et al., J. Clin. Invest., 82 (1988) 1963-1973. Dieser Effekt der PR-3 war gravierender als die Wirkung der Leukozyten-Elastase. Zahlreiche weitere Studien legen den Schluß nahe, daß die elastinolytische Aktivität der Leukozyten einen wichtigen pathogenetischen Faktor beim Lungenemphysem, beim akuten Lungenschocksyndrom, bei der Glomerulonephritis, bei rheumatoider Arthritis, bei Sepsis und bei anderen entzündlichen Erkrankungen darstellt. Freisetzung der Proteinase 3 aus neutrophilen Granulozyten bei diesen Krankheitszuständen kann durch Messung der Konzentrationen von PR-3 bzw. von PR-3-Inhibitorkomplexen im Plasma und in anderen biologischen Körperflüssigkeiten, beispielsweise in Gelenksexsudaten diagnostiziert werden. Dadurch könnte der Schweregrad entzündlicher Reaktionen beim Patienten ermittelt und die Indikation für eine zukünftige anti-enzymatische Therapie gestellt werden.

### Beispiel 1:

### Reinigung, biochemische Charakterisierung und amino-terminale Sequenzierung der Proteinase 3:

### a) Isolierung von Proteinase 3-spezifischen Autoantikörpern aus dem Serum eines Patienten mit Wegener'scher Granulomatose und Herstellung einer Affinitätssäule

Analog zur Vorgehensweise bei anderen Autoantigenen wurden zur Reinigung des ACPA-Antigens Autoantikörper aus Patientenseren eingesetzt. Dazu wurde Serum eines Patienten ausgewählt, das in der indirekten Immunfluoreszenz das typische ACPA-Muster zeigte, in dem aber keine anderen Autoantikörper nachweisbar waren. Die Immunoglobuline aus 60 ml dieses Serums wurden mit Hilfe von Ammoniumsulfat-Präzipitation (Endkonzentration: 33% Sättigung) angereichert. Die präzipitierten Immunglobuline wurden gegen PBS dialysiert und entsprechend den Herstellerangaben mit 5 mg Protein/ml Gel an CNBr-aktivierte Sepharose 4B (Pharmacia) gebunden. Verbleibende aktive Bindungsstellen wurden mit 0,2 M Glycin, pH 8, geblockt. Anschließend wurde das Gel mit 5 Zyklen von abwechselnd Kopplungspuffer (0,1 M Natriumhydrogencarbonat, pH 8,3 mit 0,5 M NaCl) und 0,1 M Azetatpuffer, pH 4 mit 0,5 M NaCl gewaschen und dann mit PBS plus 0,02 % Natriumazid gespült. Von diesem Affinitätsgel wurden 60 ml in eine 400 x 16 mm Säule gefüllt und bei 4°C gelagert.

### b) Affinitätsreinigung der Proteinase 3

Granulozyten wurden aus dem Blut von gesunden Spendern isoliert (Lüdenmann et al., J. Immunol. Methods 114, 167-174, 1988) und mit PBS plus 0,5 mM Magnesiumchlorid und 0,9 mM Kalziumchlorid auf 4 x 10⁶ Zellen/ml eingestellt. Die Granulozyten wurden dann mit 1 »g/ml Phorbolmyristat-azetat (Sigma) für 30 min bei 37°C stimuliert, um eine Degranulation zu induzieren. Durch Zentrifugation bei 500 x g wurden die Zellen abgetrennt, und der Degranulationsüberstand mit dem freigesetzten ACPA-Antigen wurde durch Sterilfiltration geklärt.

Zur Reinigung des ACPA-Antigens wurden 30 ml von 10-fach konzentriertem Degranulationsüberstand mit einer Flußgeschwindigkeit von 20 ml/Std. dreimal durch die Affinitätssäule gepumpt. Nach Waschen mit PBS (ca. 20-faches Säulenvolumen) wurde das gebundene Antigen mit Hilfe eines stufigen pH-Gradienten in Stufen von 0,5 pH-Einheiten zwischen pH 6 und pH 3,5 eluiert. Der pH-Gradient wurde mit Hilfe von 0,1 M Natrium-azetat-Puffer mit 0,5M NaCl erzeugt. Das Antigen eluierte zwischen pH 4,0 und 3,5 und wurde gegen PBS mit 0,02 % Natriumazid dialysiert.

### c) Amino-terminale Sequenzierung der Proteinase 3

Das affinitätsgereinigte Antigen (PR-3) wurde mit Hilfe eines PhastSystems (Pharmacia) elektrophoretisch aufgetrennt. Dazu wurde SDS-PAGE unter nicht-reduzierenden Bedingungen in einem 20%igen Acrylamidgel eingesetzt. Anschließend erfolgte der elektrophoretische Transfer auf eine Polyvinylidendifluorid-Membran unter Einsatz einer Standardmethode ("Semi-dry Blotting"). Mit Hilfe von Immunoblotting mit ACPA-positiven Seren von WG-Patienten wurde die Lage des ACPA-Zielantigens im Bereich eines relativen Molekulargewichtes von 38 kD lokalisiert. Parallel dazu wurde eine Blotting-Membran mit Coomassie Blau gefärbt und die Bande, die das Antigen enthielt, ausgeschnitten. Anschließend wurde die Proteinase 3 direkt auf der Membran in einem Gas-Phasen-Sequenzierer nach dem Edmann'schen Abbauverfahren amino-terminal ansequenziert. Dabei wurden die folgenden 21 amino-terminalen Aminosäuren ermittelt : I-V-G-G-H-E-A-Q-P-H-S-R-P-Y-M-A-S-L-Q-M-R.

### d) Biologische Aktivität der gereinigten Proteinase 3

Zur Untersuchung der enzymatischen Aktivität von Proteinase 3 wurde zunächst das relativ unspezifische Substrat α-Naphthylazetat eingesetzt, wobei die Freisetzung von α-Naphthol photometrisch bei 520 nm gemessen wurde. Das pH-Optimum lag mit diesem Substrat im Bereich zwischen pH 6,75 und 7,25, was belegt, daß PR-3 eine neutrale Protease ist. Die enzymatische Aktivität von PR-3 konnte durch die spezifischen Inhibitoren für Serin-Proteasen, Diisopropylfluorophosphat und Phenylmethylsulfonylfluorid, gehemmt werden. Wie aus der abgeleiteten Aminosäuresequenz ersichtlich, sind darüber hinaus die Aminosäuren, die die typische katalytische Triade bilden (His-44, Asp-91, Ser-176), vorhanden. Die spezifischen Peptidsubstrate für humane Leukozyten-Elastase (HLE) (Suc-Ala-Ala-Ala-pNA, Suc-Ala-Ala-Val-pNa und MeO-Suc-Ala-Ala-Pro-Val-pNA), für Cathepsin G (Suc-Ala-Ala-Pro-Phe-pNa) und für Trypsin (Tosyl-Arg-Methyl-ester) wurden von PR-3 nicht gespalten. Dies bestätigt, daß die PR-3-Präparation keine Beimengungen bekannter Granulozyten-Proteasen enthält und daß es sich bei der Proteinase 3 um eine neue neutrale Serin-Protease handelt.

Um zu untersuchen, ob PR-3 in der Lage ist, das biologisch relevante Substrat Elastin zu spalten, wurde PR-3, wie oben beschrieben, mit SDS-PAGE unter nicht-reduzierenden Bedingungen separiert, um eventuell noch vorhandene Spuren von Verunreinigungen abzutrennen. Das Elektrophorese-Gel wurde dann direkt auf eine Agaroseplatte gelegt, in die nach Angaben der Hersteller fein pulverisiertes, Fluoreszein-markiertes Elastin (Elastin Products Company) gegossen war. Nach einer Inkubationsdauer von 16 Stunden bei 37°C war im Bereich der Proteinase 3-Bande ein massiver Abbau der Elastin-Partikel sichtbar. Proteinase 3 hat also elastinolytische Aktivität.

Um den Einfluß von Enzyminhibitoren auf die elastinolytische Aktivität von Proteinase 3 zu untersuchen, wurden in die Elastin-Platten Löcher gestanzt, PR-3 mit bzw. ohne Inhibitor eingefüllt und wie oben beschrieben inkubiert Die Spaltung von Elastin ist als Lysezone um die Löcher herum sichtbar und kann durch Berechnung der Fläche der Lysezone grob quantifiziert werden. Die Zugabe des biologisch relevanten menschlichen Enzyminhibitors α-1-Protease-Inhibitor zu PR-3 inhibierte die elastinolytische Aktivität der PR-3 vollständig. Die Wirkung der Autoantikörper (ACPA) auf PR-3 wurde mit dem gleichen Versuchsansatz untersucht. Um eine Interferenz der Protease-Inhibitoren des Serums zu verhindern, wurde mit Hilfe von Protein G-Chromatographie die Immunglobulin-G (IgG)-Fraktion aus den Patientenseren isoliert Patienten-IgGs wurden zusammen mit PR-3 eine Stunde lang bei Raumtemperatur inkubiert Anschließend wurde das Reaktionsgemisch in Elastin-Platten gefüllt. Bei fast allen Patienten mit Wegener'scher Granulomatose inhibierten die Immunglobuline die elastinolytische Aktivität der Proteinase 3.

### Beispiel 2:

### Klonierung der cDNA für Proteinase 3

### a) Herstellung von synthetischen Oligonukleotid-Primern für die Polymerase-Ketten-Reaktion

Unter der Annahme, daß die cDNA-Sequenz der Proteinase 3 der publizierten cDNA-Sequenz des Myeloblastins (Bories et al., Cell 59, 959-968, 1989) sehr ähnlich ist, letztendlich jedoch von ihr an gewissen Positionen abweicht, wurde zunächst die 5'-cDNA-Sequenz des Myeloblastins einer genauen Analyse unterzogen und mit Hilfe der beiden Oligonukleotide JT 85 und JT 88 und der thermostabilen TAQ-DNA-Polymerase aus totaler komplementärer DNA der U937-Zell-Line amplifiziert. Das Oligonukleotid JT 88 (28mer) hat die Sequenz 5'-CCGAATTC TGAGCGGTGCTGCCCGAGCT-3' und ist von der 9. Base an mit der Position -66 bis -47 der Myeloblastin-Sequenz identisch (Bories et al., Cell 59, 959-968, 1989). JT 85 hat die Sequenz 5'-CCGAATT CAGAAGTGGCTGCCCGGGTT-3' und ist von der 8. Base an mit dem von Position +25 bis +44 komplementären Basenstrang der Myeloblastin-cDNA-Sequenz identisch. Das Oligonukleotid JT 90 hat die Sequenz 5'-CCGAATTC GAGGGTTTGGAGCCAGG CTC-3' und ist ab der 9. Base mit dem von Position 749 bis 768 komplementären Basenstrang der PR-3-cDNA-Sequenz (**Abb. 3**) identisch. Die zusätzlichen 7 bzw. 8 Nukleotidbasen an den 5'-Enden enthalten *Eco* RI-Restriktionsschnittstellen und erleichtern das Subklonieren amplifizierter cDNA nach Verdauung mit dem Restriktionsenzym *Eco* RI.

### b) cDNA-Synthese von U937 mRNA

Totale mRNA von U937 Zellen wurde nach den Vorschriften des Stratagene RNA-Isolationskits isoliert. Daraus wurde Poly(A⁺)-RNA durch Affinitätschromatographie an Oligo(dT)-Cellulose gewonnen. Der erste Strang der cDNA wurde mit Hilfe der reversen Transkriptase des Avian Myeloblastosis Virus (AMV) und eines Oligo (dT)-Primers der Kettenlänge 12 bis 18 in dem mit dem Enzym mitgelieferten Puffersystem der Firma BRL synthetisiert. Die Template-RNA wurde durch Alkali-Behandlung hydrolysiert, die einzelsträngige cDNA danach aufgereinigt und gefällt. Ungefähr 20 ng dieser cDNA wurden für eine Polymerase-Ketten-Reaktion (PCR) eingesetzt.

### c) PCR unter Verwendung der Oligonukleotide JT 85 und JT 88

Die PCR-Technik wurde nach der Arbeitsanleitung der Firma Perkin-Elmer mit dem empfohlenen Puffersystem durchgeführt. Die Desoxynukleotid-Konzentrationen betrugen jeweils 200 »M, die Konzentration der beiden synthetischen Oligonukleotide 0,2 »M. Die Magnesium-Konzentration betrug in den drei verschiedenen Ansätzen 1,5 mM, 1,7 mM und 1,9 mM. Mit dem folgenden Temperaturprogramm konnte die oben gestellte Aufgabe gelöst werden: zwei Minuten Denaturierung der DNA bei 93°C, 2 min Hybridisierung der Primer bei 59°C und drei Minuten Polymerisierung bei 72°C mit Hilfe der hitzestabilen TAQ-DNA-Polymerase. Die geschilderten Reaktionen (ein Reaktionszyklus) wurden 27 mal hintereinander wiederholt. In jedem Zyklus wird die Menge des gesuchten DNA-Abschnittes verdoppelt. Das Produkt wurde durch dreimalige Fällung in 2M Ammoniumacetat und in Anwesenheit von linearem Acrylamid aufgereinigt.

### d) PCR unter Verwendung der Oligonukleotide JT 88 und JT 90

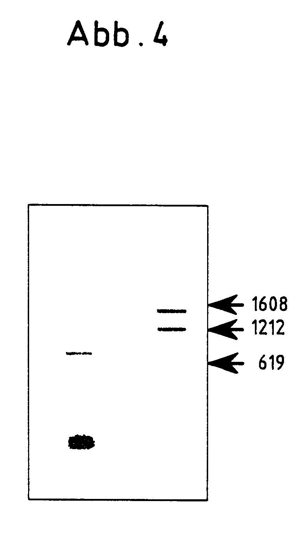

Fur die Klonierung der kodierenden Region der Proteinase 3 wurden die beiden Oligonukleotide JT 88 und JT 90 verwendet. Die Reaktionsbedingungen wurden ähnlich wie unter c) gewählt, mit den Unterschied, daß die Hybridisierung der Primer bei 55°C erfolgte, die Magnesiumkonzentration 1.7 mM betrug und nur 25 Amplifikationszyklen benutzt wurden. Das amplifizierte Reaktionsprodukt hat unter Berücksichtigung der beiden zusätzlichen Nukleotide in der 5'-Region und der 18 Nukleotide an den beiden Enden der PCR-Primer eine Gesamtlänge von 784 Basenpaaren (siehe **Abb. 4**). Nach Restriktionsverdauung mit *Eco* RI wurde das PCR-Produkt durch Agarose-Gel-Elektrophorese (1,2%ig) aufgetrennt und aus dem Gel eluiert. Die cDNA wurde in einen *Eco* RI geschnittenen, dephosphorylierten Vektor, M13mp18/BAP (Pharmacia) subkloniert.

### e) cDNA-Basensequenzbestimmung

Nukleotidsequenzen wurden nach den Protokollen des Sequenase-Kits (United States Biochemical Corporation) bestimmt. Die Nukleotidbasensequenz des 5'-Endes der Proteinase 3 (siehe unter c) wurde mehrmals und bei verschiedenen Klonen bestimmt. Heterogenität oder Polymorphismen bei den einzelnen cDNA-Klonen wurden nicht beobachtet (**Abb. 2**).

Die verschiedenen M13-Klone mit der kompletten cDNA-Insertion für Proteinase 3 (nach Vorschrift d hergestellt) werden zur Zeit mit Hilfe von Oligonukleotiden, die von der Proteinase-3-Sequenz (**Abb. 3**) abgeleitet sind, sequenziert, um Klonierungs- oder Amplifizierungsartefakte, die in vereinzelten Fällen auftreten können, auszuschließen.

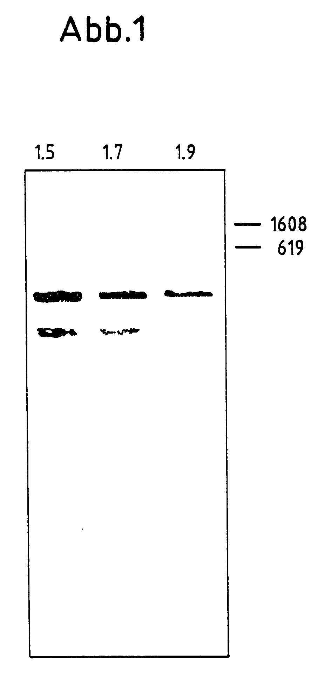

**Abbildung 1** zeigt die PCR-Produkte, die mit Hilfe der beiden Oligonukleotide JT 85 und JT 88 und gesamter U937 cDNA bei verschiedenen Magnesium-Konzentrationen (oberer Bildrand in mM) amplifiziert wurden. Die Länge des amplifizierten cDNA-Fragmentes beträgt insgesamt 127 Basenpaare.

**Abbildung 2** Nukleotid- und Aminosäure-Sequenzen der Proteinase 3 (PR-3), des Zielantigens von Autoantikörpern bei der Wegener'schen Granulomatose. PR-3 und die humane Leukozyten-Elastase (HLE) sind in der zweiten und dritten Zeile unterhalb der Nukleotidsequenz miteinander verglichen. Zwecks optimaler Angleichung der HLE- und PR-3-Sequenzen wurden drei Lücken in die Sequenz von HLE eingefügt (-). Die Nummerierung der PR-3-Sequenz beginnt mit der ersten Aminosäure, die bei der Aminosäure-Sequenzierung der gereinigten Proteinase 3 gefunden wurde (unterstrichener Teil der Aminosäure-Sequenz). Insgesamt wurden 21 Aminosäuren auf Proteinebene bestimmt. Die dargestellte cDNA wurde durch PCR-Amplifikation unter Verwendung der Oligonukleotide JT 85 und JT 88 (Nukleotidsequenz unterstrichen) kloniert und sequenziert. Die beiden Pfeile oberhalb der cDNA-Sequenz zeigen auf die beiden Nukleotide, die in der publizierten cDNA-Sequenz des Myeloblastins fehlen. Der Pfeil unterhalb der beiden Proteinsequenzen markiert die Spaltstelle für das Enzym Signalpeptidase. Die beiden folgenden Aminosäuren Ala-Glu bilden ein kurzes Propeptid ähnlich wie bei der Leukozyten-Elastase. Die aktive Form der Proteinase 3 entsteht durch Abspaltung des Signalpeptides und des Propeptides während der intrazellulären Biosynthese.

**Abbildung 3** zeigt die Nukleotidbasensequenz der cDNA für Proteinase 3 und die davon abgeleitete Proteinsequenz. Die ersten 6 translatierten Aminosäuren bilden einen Teil des Signalpeptides, die folgenden beiden Aminosäuren das Propeptid (Nummer -8 bis -1). Die nachfolgenden Aminosäuren (1 bis 229) stellen die komplette Proteinsequenz der aktivierten, granulär gespeicherten Proteinase 3 dar.

**Abbildung 4** zeigt die PCR-Produkte, die mit Hilfe der beiden Oligonukleotide JT 88 und JT 90 aus gesamter U937 cDNA bei einer Magnesium-Konzentration von 1.7 mM amplifiziert wurden (linke Spur), rechts daneben Molekulargewichtsmarker, deren Größe in Basenpaare angegeben sind. Die Länge des amplifizierten cDNA-Fragmentes beträgt einschließlich der 5'-terminal angefügten *Eco* RI Restriktionsschnittstellen 784 Basenpaare. Dieses Fragment wurde aus dem Agarose-Gel eluiert und nach Restriktionsverdauung mit *Eco* RI in M13mp18 subkloniert. Es enthält die gesamte genetische Information für die aktive, sezernierte Form der Proteinase 3.

## Patentansprüche

1. sDNA, kodierend ein Enzym mit Serinprotease-Aktivität (Proteinase 3) und *gekennzeichnet* durch (in Leserichtung)
(a) fakultative Kodons für ein Signalpeptid von Serinprotease,
(b) 2 fakultative Kodons für ein Propeptid von Serinprotease und
(c) 229 Kodons für das prozessierte Enzym mit Serinprotease-Aktivität (1 bis 229) gemäß Figur 3, wobei beim Merkmal (c)
- 1 bis 10 oder bis 20 Kodons gemäß Figur 3 gegen andere Kodons ausgetauscht sein können und/oder
- 1 bis 10 oder bis 20 Kodons fehlen oder hinzugefügt sein können.

2. sDNA nach Anspruch 1, dadurch *gekennzeichnet*, daß beim Merkmal (c) am 5'-Ende für das prozessierte Enzym mit Serinprotease-Aktivität 1 bis 10 oder bis 20 Kodons hinzugefügt sein können.

3. sDNA nach Anspruch 1 oder 2, dadurch *gekennzeichnet*, daß es sich bei den Kodons für das Signalpeptid (-8 bis -3) um die Kodons gemäß Figur 3 oder eine vom 5'-terminalen Ende her um ein oder mehrere Kodons verkürzte DNA-Sequenz handelt.

4. sDNA nach einem der vorhergehenden Ansprüche, dadurch *gekennzeichnet*, daß es sich bei den 2 Kodons für ein Propeptid von Serinprotease (Proteinase 3) um die Kodons handelt, die den Aminosäuresequenz-Positionen -2 bis -1 in Figur 3 entsprechen.

5. sDNA nach einem der vorhergehenden Ansprüche, dadurch *gekennzeichnet*, daß ein oder mehrere Kodons gemäß Figur 3 durch Kodons ersetzt sind, die dieselbe Aminosäure kodieren.

6. sDNA nach einem der vorhergehenden Ansprüche, ergänzt um einen komplementären Strang (cDNA).

7. Verwendung einer cDNA gemäß Anspruch 6 für oder in einem Expressionsvektor zur Expression eines Enzyms mit Serinprotease-Aktivität in Mikroorganismen, insbesondere in E. coli.

8. Verwendung einer cDNA gemäß Anspruch 6 für oder in einer eukaryontischen Zell-Linie zur Expression eines Enzyms mit Serinprotease-Aktivität.

## Claims

1. ssDNA encoding an enzyme having serine protease activity (proteinase 3) and characterized by (in the direction of reading)
(a) optional codons for a signal peptide of serine protease,
(b) 2 optional codons for a propeptide of serine protease and
(c) 229 codons for the processed enzyme having serine protease activity (1 to 229) according to Figure 3, where in feature (c)
- 1 to 10 or up to 20 codons according to Figure 3 may be exchanged for other codons and/or
- 1 to 10 or up to 20 codons may be missing or added.

2. ssDNA according to Claim 1, characterized in that in feature (c) 1 to 10 or up to 20 codons may be added at the 5'-end for the processed enzyme having serine protease activity.

3. ssDNA according to Claim 1 or 2, characterized in that the codons for the signal peptide (-8 to -3) are the codons according to Figure 3 or are a DNA sequence shortened from the 5'-terminal end by one or more codons.

4. ssDNA according to one of the preceding claims, characterized in that the 2 codons for a propeptide of serine protease (proteinase 3) are the codons which correspond to the amino acid sequence positions -2 to -1 in Figure 3.

5. ssDNA according to one of the preceding claims, characterized in that one or more codons according to Figure 3 are replaced by codons which encode the same amino acid.

6. ssDNA according to one of the preceding claims, supplemented by a complementary strand (cDNA).

7. Use of a cDNA according to Claim 6 for or in an expression vector for expressing an enzyme having serine protease activity in microorganisms, in particular in E. coli.

8. Use of a cDNA according to Claim 6 for or in a eukaryotic cell line for expressing an enzyme having serine protease activity.

## Revendications

1. ADNs codant pour une enzyme ayant l'activité de sérine protéase (protéinase 3) est *caractérisé* par (dans le sens de la lecture)
(a) des codons facultatifs pour un signal peptide de sérine protéase,
(b) 2 codons facultatifs pour un propeptide de sérine protéase et
(c) 229 codons pour l'enzyme mature ayant l'activité de sérine protéase (1 à 229) selon la figure 3, dans le cas de la caractéristique (c)
- 1 à 10 ou jusqu'à 20 codons selon la figure 3 pouvant être échangés contre d'autres codons et/ou
- 1 à 10 ou jusqu'à 20 codons étant manquants ou pouvant être ajoutés.

2. ADNs selon la revendication 1, *caractérisé* en ce que dans le cas de la caractéristique (c) on peut, à l'extrémité 5' de l'enzyme mature avec une activité de sérine protéinase, ajouter de 1 à 10 ou jusqu'à 20 codons.

3. ADNs selon la revendication 1 où 2, *caractérisé* en ce que dans le cas des codons pour le peptide signal (-8 à -3), il s'agit des codons selon la figure 3 ou d'une séquence d'ADN raccourcie d'un ou plusieurs codons à l'extrémité 5'.

4. ADNs selon l'une quelconque des revendications précédentes, *caractérisé* en ce que dans le cas des deux codons pour un propeptide de sérine protéase (protéinase 3) il s'agit des codons qui correspondent aux positions -2 à -1 de la séquence d'acides aminés illustrée à la figure 3.

5. ADNs selon l'une quelconque des revendications précédentes, *caractérisé* en ce qu'un ou plusieurs codons selon la figure 3 sont remplacés par des codons qui codent pour le même acide aminé.

6. ADNs selon l'une quelconque des revendications précédentes complété d'un brin complémentaire (ADNc).

7. Utilisation d'un ADNc selon la revendication 6 pour ou dans un vecteur d'expression pour exprimer une enzyme ayant une activité de sérine protéase dans des micro-organismes, plus particulièrement dans l'*E. coli*.

8. Utilisation d'un ADNc selon la revendication 6 pour exprimer ou pour exprimer dans une lignée cellulaire eucaryote une enzyme ayant une activité de sérine protéase.
